# EUROPEAN PATENT APPLICATION

(11) **EP 2 829 608 A1**
(43) Date of publication of application: **28.01.2015**
(21) Application number: 13177678.3
(22) Date of filing: 23.07.2013
(51) Int. Cl.: C12N 15/113, C12N 5/071, C12N 5/16

(54) **Method for recombinant protein production in mammalian cells**

(71) Applicant: Universität Bielefeld, 33615 Bielefeld (DE)
(72) Inventor: Krämer, Oliver, 59602 Rüthen (DE); Klausing, Sandra, 33615 Bielefeld (DE); Noll, Thomas, 33615 Bielefeld (DE)
(74) Representative: Viering, Jentschura & Partner Patent- und Rechtsanwälte

(57) **Abstract**

This invention relates to methods for the recombinant expression of a protein of interest in a mammalian host cell, use of an shRNA or an siRNA directed against the Galectin-1 gene for increasing the expression of a protein of interest in a mammalian host cell and kits comprising said shRNA or an siRNA and a CHO cell.

SEQUENCE LISTING

<110>  Universität Bielefeld

<120>  METHOD FOR RECOMBINANT PROTEIN PRODUCTION IN MAMMALIAN CELLS

<130>  P52703

<160>  20

<170>  PatentIn version 3.5

<210>  1
<211>  408
<212>  DNA
<213>  Cricetulus griseus

<400>  1
atggcctgtg gtctggtcgc aagcaacctg aatctcaaac ctggggagtg tctcaaagtt      60

cggggcgagg tggcccctga cgccaagagc tttgtgctga acctggggaa agacagcaac     120

aacctgtgcc tgcacttcaa cccccgtttc aacgcccatg gggacgccaa cactatcgtg     180

tgcaacagca aggataacgg agcctggggg actgagcacc gggagcctgc cttccccttc     240

cagcctggaa gcactgtgga ggtatgcatc acctttgacc aggctgacct gaccatcaag     300

ctgccagatg ggcatgagtt caagttccct aaccgtctca acatggaggc catcaactac     360

atggcggcag acggcgactt caagatcaag tgtgtggcct ttgagtga                  408

<210>  2
<211>  135
<212>  PRT
<213>  Cricetulus griseus

<400>  2

Met Ala Cys Gly Leu Val Ala Ser Asn Leu Asn Leu Lys Pro Gly Glu
1               5                   10                  15

Cys Leu Lys Val Arg Gly Glu Val Ala Pro Asp Ala Lys Ser Phe Val
            20                  25                  30

Leu Asn Leu Gly Lys Asp Ser Asn Asn Leu Cys Leu His Phe Asn Pro
        35                  40                  45

Arg Phe Asn Ala His Gly Asp Ala Asn Thr Ile Val Cys Asn Ser Lys
    50                  55                  60

Asp Asn Gly Ala Trp Gly Thr Glu His Arg Glu Pro Ala Phe Pro Phe
65                  70                  75                  80

Gln Pro Gly Ser Thr Val Glu Val Cys Ile Thr Phe Asp Gln Ala Asp
                85                  90                  95

Leu Thr Ile Lys Leu Pro Asp Gly His Glu Phe Lys Phe Pro Asn Arg
            100                 105                 110

Leu Asn Met Glu Ala Ile Asn Tyr Met Ala Ala Asp Gly Asp Phe Lys
        115                 120                 125

Ile Lys Cys Val Ala Phe Glu
    130                 135

<210>  3
<211>  54
<212>  RNA
<213>  Artificial Sequence

<220>
<223>  shRNA Galectin-1

<400>  3
gucgcaagca accugaaucu uucaagagaa gauucagguu gcuugcgauu uuuu          54

<210>  4
<211>  20
<212>  RNA
<213>  Artificial Sequence

<220>
<223>  siRNA Galectin-1

<400>  4
gucgcaagca accugaaucu                                                20

<210>  5
<211>  20
<212>  DNA
<213>  Artificial Sequence

<220>
<223>  RT-PCR primer fwd Galectin-1

<400>  5
ggggagtgtc tcaaagttcg                                                20

<210>  6
<211>  19
<212>  DNA
<213>  Artificial Sequence

<220>
<223>  RT-PCR primer rev Galectin-1

<400>  6
ggttgaagtg caggcacag                                                 19

<210>  7
<211>  52

<212>  RNA
<213>  Artificial Sequence

<220>
<223>  shRNA Set

<400>  7
gaugaaggug aagaagaugu ucaagagaca ucuucuucac cuucaucuuu uu          52


<210>  8
<211>  19
<212>  RNA
<213>  Artificial Sequence

<220>
<223>  siRNA Set

<400>  8
gaugaaggug aagaagaug                                               19


<210>  9
<211>  26
<212>  DNA
<213>  Artificial Sequence

<220>
<223>  RT-PCR primer fwd Set

<400>  9
ttcagaagag gtcagaattg atcgcc                                       26


<210>  10
<211>  22
<212>  DNA
<213>  Artificial Sequence

<220>
<223>  RT-PCR primer rev Set

<400>  10
tcgtcttcct ccccaagcag gg                                           22


<210>  11
<211>  52
<212>  RNA
<213>  Artificial Sequence

<220>
<223>  shRNA Bad

<400>  11
ggaugagcga ugaguuugau ucaagagauc aaacucaucg cucauccuuu uu          52


<210>  12
<211>  19
<212>  RNA
<213>  Artificial Sequence

<220>
<223>  siRNA Bad

<400>  12
ggaugagcga ugaguuuga                                                    19


<210>  13
<211>  21
<212>  DNA
<213>  Artificial Sequence

<220>
<223>  RT-PCR primer fwd Bad

<400>  13
ctggctcctg cacacgccct a                                                 21


<210>  14
<211>  24
<212>  DNA
<213>  Artificial Sequence

<220>
<223>  RT-PCR primer rev Bad

<400>  14
acatactctg ggctgctggt ctcc                                              24


<210>  15
<211>  18
<212>  DNA
<213>  Artificial Sequence

<220>
<223>  RT-PCR primer fwd Aktin

<400>  15
caccctgtgc tgctcacc                                                     18


<210>  16
<211>  18
<212>  DNA
<213>  Artificial Sequence

<220>
<223>  RT-PCR primer rev Aktin

<400>  16
cgtacatggc tggggtgt                                                     18


<210>  17
<211>  18
<212>  DNA
<213>  Artificial Sequence

<220>
<223>  RT-PCR primer fwd Vezatin

<400>  17
ttaaggagct ggggcttg                                                    18


<210>  18
<211>  18
<212>  DNA
<213>  Artificial Sequence

<220>
<223>  RT-PCR primer rev Vezatin

<400>  18
gtgccaccca gagttgga                                                    18


<210>  19
<211>  20
<212>  DNA
<213>  Artificial Sequence

<220>
<223>  RT-PCR primer fwd Gapdh

<400>  19
ccaggtggtc tcctctgact                                                  20


<210>  20
<211>  22
<212>  DNA
<213>  Artificial Sequence

<220>
<223>  RT-PCR primer rev Gapdh

<400>  20
tcgtcttcct ccccaagcag gg                                               22

## Description

### FIELD OF THE INVENTION

The present invention lies in the field of molecular biology and relates to enhanced recombinant protein expression in mammalian cells. The present invention also relates to use of shRNA or siRNA to facilitate increased expression of a protein of interest and a kit comprising said inhibitory RNAs and mammalian host cells.

### BACKGROUND OF THE INVENTION

Among mammalian expression systems, the Chinese Hamster ovary (CHO) cell mammalian expression system is widely used for production of recombinant protein. Apart from lymphoid cell pools such as hybridoma cell pools, it is one of the few cell types allowing for simple and efficient high-density suspension batch culture of animal cells. Furthermore, they allow for very high product yields and are comparatively robust to metabolic stresses whereas lymphoid cells are more difficult to culture at an industrial scale. Given considerable cost of production, it is of utmost importance to maximize the yield of recombinant protein per bioreactor run. Choice of culture medium composition and bioreactor design and operation are parameters that impact yield but are quite complex to optimize. Changing the expression of a protein of interest at the single cell level is a more predictable approach to enhance protein yield. Incremental increases at the single cell level will translate into considerable improvements of product yield in high-density batch or fed-batch culture showing stationary phase gene expression at cell densities in the range of 10⁶ to 10⁷ cells/ml.

US5,866,359 describes a method of enhancing expression from an already strong hCMV promoter in CHO and NSO cells by co-expressing adenoviral E1A protein from a weak promoter. E1A is a multifunctional transcription factor which may act on cell cycle regulation and has both independent transcriptional activating and repressing functional domains. The finetuning of E1A expression to appropriate low level expression is crucial for success of the co-expression approach in order to achieve the ideal balance in between gene transactivation whilst avoiding any negative impact on cell cycle progression.

As a disadvantage, apart from careful choice of the promoter driving E1A expression, this system blocks part of the protein synthesis capacity of the cell with E1A expression rather than expressing the recombinant protein of interest.

WO 95/17516 describes use of the murine immunoglobulin gamma 2A locus for targeting an expression vector construct to a highly active gene locus in lymphoid cells of the B-cell poolage, e.g. widely used NSO myeloma cells. NSO cells essentially are a tumor cell pool of murine plasma or B-cells. Only in B-cells, the chromatin harboring the immunoglobulin loci is in its fully active, open state, allowing for high transcriptional activity of native immunoglobulin promoters or recombinant expression constructs integrated into those gene loci.

However, due to the principle of homologous recombination, the targeting sequence will target efficiently in murine cell pools only matching the sequence of the gamma 2 A targeting sequence harboring a recombinatorial hot spot; for high level expression, the gamma 2A locus region must be a transcriptionally active genomic region, limiting its effectiveness for homologous recombination to B-cell types.

WO 2007/123489 A1 describes overexpression of heat shock proteins in CHO cells for enhancing recombinant expression of a protein of interest.

In such methods, however, due to the high expression and sticky nature of the heat shock proteins, the protein of interest may be contaminated with the heat shock protein. Thus, purification of the protein of interest becomes more expensive and time consuming.

Hence, there is need in the art for methods and materials that allow improving recombinant protein expression in mammalian cells, in particular recombinant protein expression in CHO cells.

It is an object of the present invention to provide an expression system for protein expression in CHO cells which allows for enhanced expression from a standard promoter and does not involve overexpression of additional factors other than the protein of interest itself. This aim is surprisingly achieved by silencing the Galectin-1 gene, preferably via RNAi, or by inhibiting the activity of the Galectin-1 gene product.

The Galectin-1 (Lgals-1) gene encodes a protein that is 135 amino acids in length and highly conserved across species. The identification references of Galectin-1 proteins originating from different species are provided in the following: human: NCBI reference sequence: NP_002296.1; mouse: NCBI reference sequence: NP_032521.1; rat: NCBI reference sequence: NP_063969.1; Chinese hamster: GenBank: EGV94322.1. Galectin-1 can be found in the nucleus, the cytoplasm, the cell surface and in the extracellular space. Galectins in general lack a traditional signal sequence, but are still secreted across the plasma membrane. This non-traditional secretion requires a functional glycan binding site. Galectin-1 contains a single carbohydrate recognition domain through which it can bind glycans both as a monomer and as a homodimer. The nucleic acid sequence of Galectin-1 of *Cricetulus griseus* is set forth in SEQ ID NO:1. The corresponding protein sequence is set forth by SEQ ID NO:2.

### SUMMARY OF THE INVENTION

The present invention is based on the inventors' finding that a method comprising inhibiting the expression of the Galectin-1 gene or the activity of the Galectin-1 gene product in a mammalian host cell increases recombinant expression of a protein of interest.

In a first aspect, the present invention is thus directed to a method for the recombinant expression of a protein of interest in a mammalian host cell, wherein the method comprises culturing the mammalian host cell comprising a nucleic acid sequence encoding the protein of interest under conditions suitable for recombinant expression of the protein of interest, and inhibiting the expression of the Galectin-1 gene or the activity of the Galectin-1 gene product in the mammalian host cell.

In various embodiments of this first aspect of the invention, the expression of the Galectin-1 gene is inhibited by RNAi.

In preferred embodiments of the invention, the RNAi is shRNA or siRNA.

In more preferred embodiments of the invention, the shRNA comprises the nucleic acid sequence set forth in SEQ ID NO:3.

In some embodiments, the siRNA comprises the nucleic acid sequence set forth in SEQ ID NO:4.

In various embodiments, the mammalian host cell is a Chinese hamster ovarian (CHO) cell.

In other various embodiments of the invention, the mammalian host cell is a Chinese hamster ovarian (CHO) DP-12 cell (ATCC CRL-12445).

In various embodiments, the protein of interest is an antibody. In preferred embodiments, the antibody is the monoclonal murine 6G4.2.5 antibody (ATCC-HB-11722).

In other various embodiments of the invention, the mammalian host cell is cultured by fed-batch process.

In still further embodiments of the first aspect, a nucleic acid sequence encoding the RNAi is stably integrated into the genome of the mammalian host cell.

In a second aspect, the invention relates to a kit comprising a CHO cell and an shRNA or an siRNA, wherein the shRNA or the siRNA inhibit the expression of the Galectin-1.

In preferred embodiments of this second aspect of the invention, the shRNA comprises the nucleic acid sequence set forth in SEQ ID NO:3.

In other preferred embodiments, the siRNA comprises the nucleic acid sequence set forth in SEQ ID NO:4.

In a further aspect, the invention is directed to a use of an shRNA or an siRNA directed against the Galectin-1 gene for increasing the expression of a protein of interest in a mammalian host cell.

In preferred embodiments of the third aspect, the shRNA comprises the nucleic acid sequence set forth in SEQ ID NO:3 or the siRNA comprises the nucleic acid sequence set forth in SEQ ID NO:4.

### BRIEF DESCRIPTION OF THE DRAWINGS

The invention will be better understood with reference to the detailed description when considered in conjunction with the non-limiting examples and the accompanying drawings.

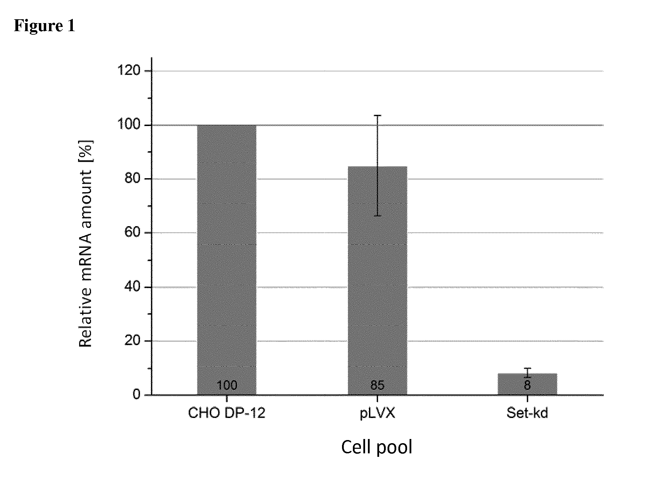

**Figure 1** shows quantitative real-time PCR measurements of Set mRNA obtained from pLVX cells and Set knockdown (Set-kd) cells compared to the amount of Set-mRNA in CHO DP-12 cells. The Set mRNA amount transcribed in Set-kd cells was about 8 % of the Set mRNA amount found in CHO DP-12 cells.

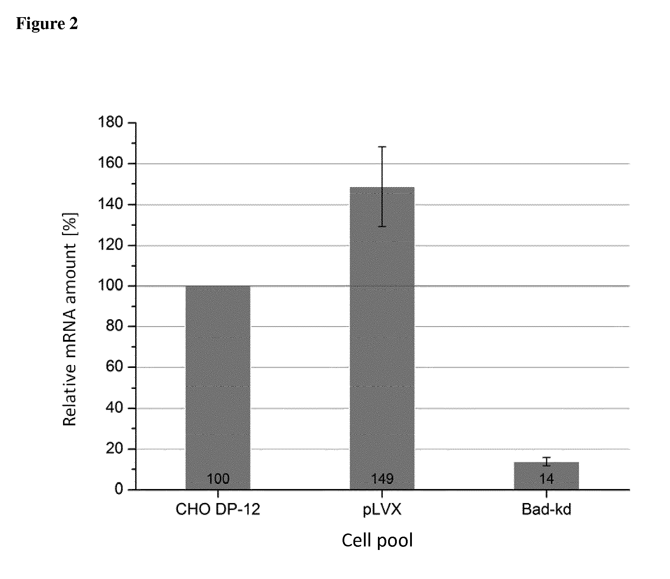

**Figure 2** shows quantitative real-time PCR measurements of Bad mRNA obtained from pLVX cells and Bad knockdown (Bad-kd) cells compared to the amount of Bad-mRNA in CHO DP-12 cells. The Bad mRNA amount transcribed in Bad-kd cells was about 14 % of the Bad mRNA amount found in CHO DP-12 cells.

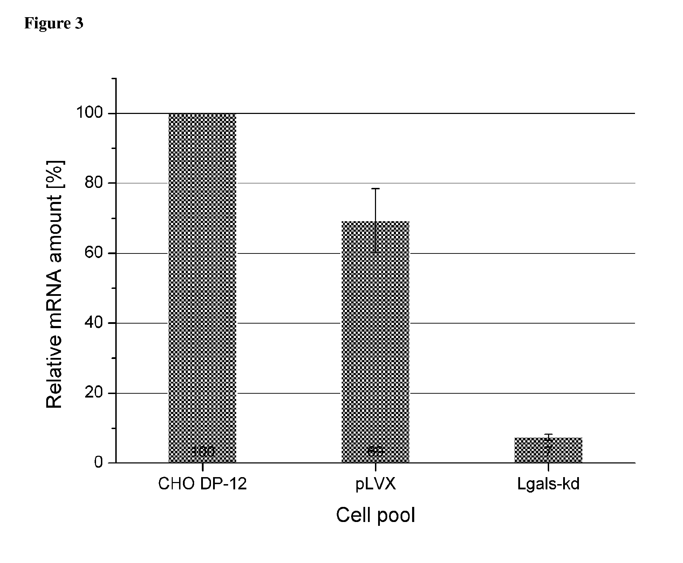

**Figure 3** shows quantitative real-time PCR measurements of Galectin-1 (Lgals-1) mRNA obtained from pLVX cells and Lgals-1 knockdown (Lgals-1-kd) cells compared to the amount of Lgals-1 mRNA in CHO DP-12 cells. The Lgals-1 mRNA amount transcribed in Lgals-1-kd cells was about 7 % of the Lgals-1 mRNA amount found in CHO DP-12 cells transfected with scrambled RNAi.

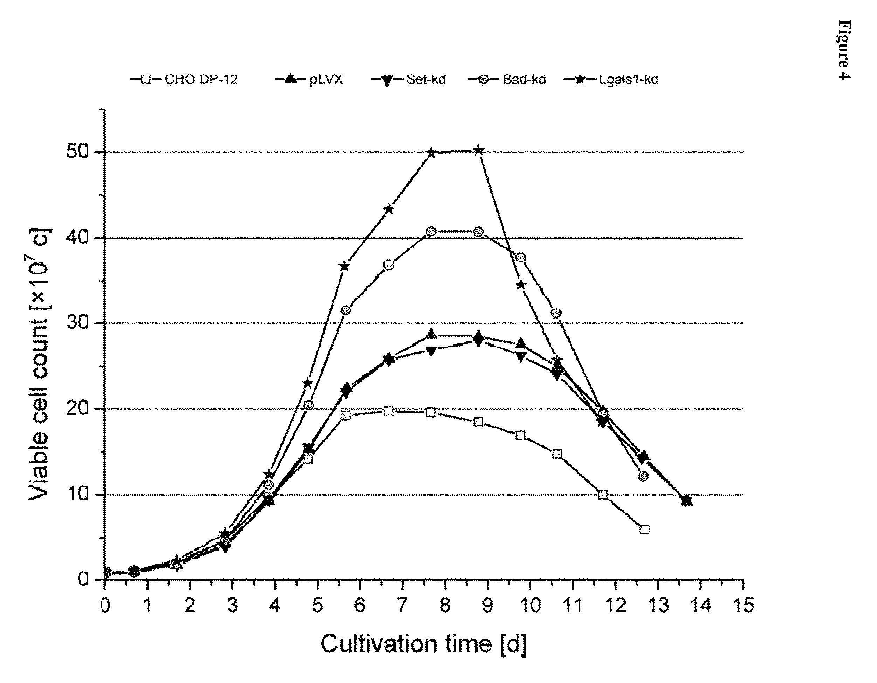

**Figure 4** shows the number of viable cells during fed-batch cultivation for CHO DP-12 cells, the pLVX cell pool, the Set-kd cell pool, the Bad-kd cell pool and the Lgals1-kd cell pool over a range of up to 14 days.

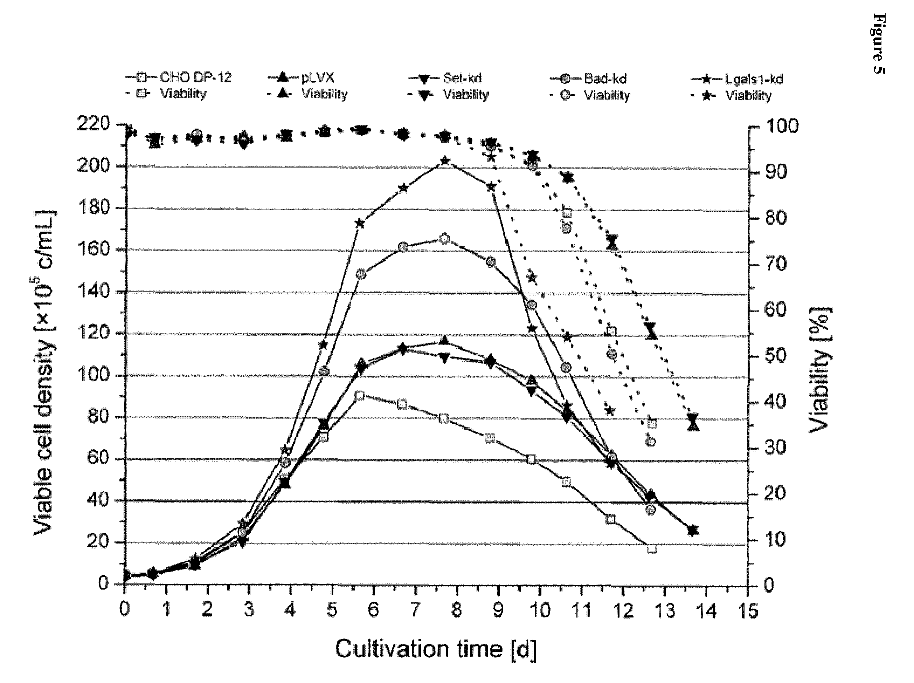

**Figure 5** shows shaker-fed-batch cultivations of CHO DP-12 cells, pLVX cells, Set-kd cells, Bad-kd cells and Lgals1-kd cells. Depicted are the density of viable cells (see continuous lines) and the viability (see dashed lines) during the cultivation of up to 14 days. The highest density of viable cells was achieved by Lgals-1-kd cells followed by Bad-kd cells. The vector control cells (pLVX cells) and Set-kd cells almost grew identical and yielded in increased densities of living cells over the reference culture (CHO DP-12 cells).

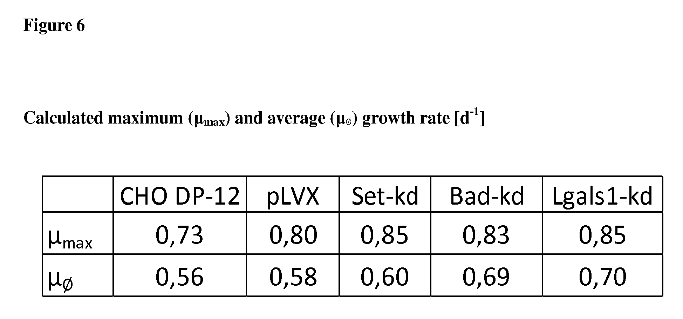

**Figure 6** shows results for the maximum (µₘₐₓ) and average (µ_{Ø}) growth rate [d⁻¹] of the reference culture and the indicated cell pools during fed-batch cultivation. µₘₐₓ represents the rate of growth observed if none of the nutrients are limited. The calculation of µ_{Ø} relies solely on data points of the exponential growth phase which was observed between days 1.7 and 5.6.

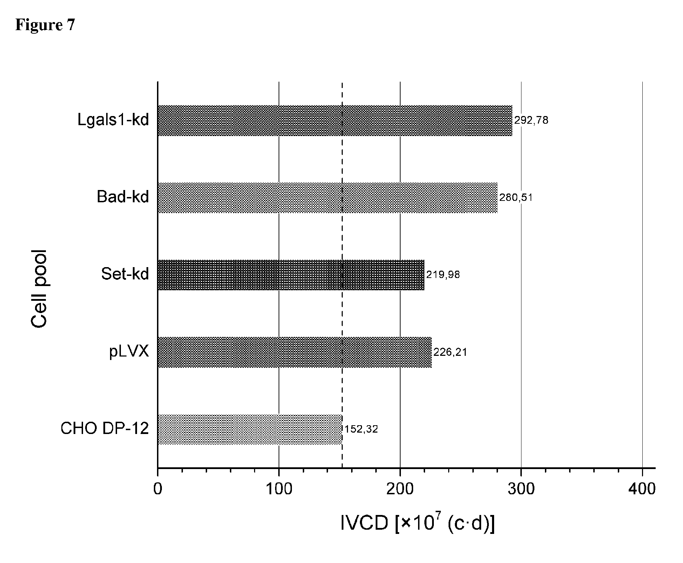

**Figure 7** shows cell-time integrals of the indicated fed-batch cultivated cells. The dashed line indicates the cell-time integral of the reference culture (CHO DP-12 cells). The vector control cells (pLVX cells) and Set-kd cells have almost identical cell-time integrals and showed 48.5 % and 44.4 % higher yield of viable cells compared to the reference culture. The highest yield of viable cells was observed for Lgals1-kd cells followed by Bad-kd cells. Their cell-time integrals have been increased for 92.2 % and 84.2 % compared to CHO DP-12 cells. Note that the calculation of the cell-time integral considers changes of the culture volume and thus the dimension of the cell-time integral is x10⁷ (c·d).

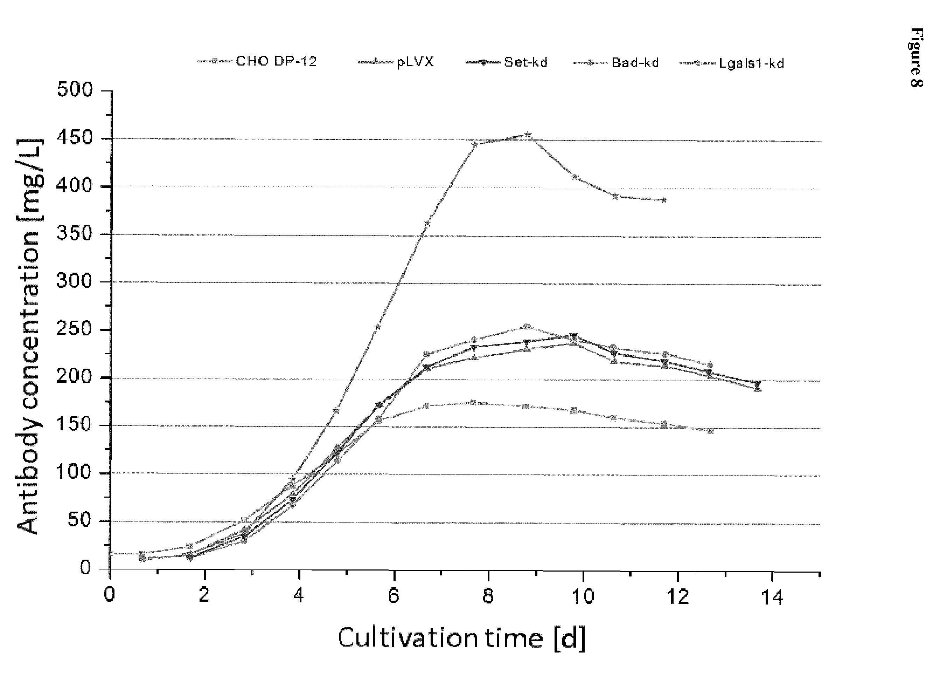

**Figure 8** shows the antibody concentrations produced by fed-batch cultivation of the indicated cells. All cell pools generate higher product titers than the reference culture (CHO DP-12 cells). However, only the Lgals1-kd cell pool produces a product titer that is higher than the titer of the vector control cells (pLVX cells).

### DETAILED DESCRIPTION OF THE INVENTION

The terms used herein have, unless explicitly stated otherwise, the following meanings.

The term "expression", as used herein, relates to a process in which information from a gene is used for the synthesis of a gene product. In cell-based expression systems the expression comprises transcription and translation steps. The expression may be induced by an inductor such as tetracycline or may be constitutive. Inducible and constitutive promoters are known in the art.

The term "recombinant expression", as used herein, relates to transcription and translation of an exogenous gene in a host organism. Exogenous DNA refers to any deoxyribonucleic acid that originates outside of the host cell. The exogenous DNA may be integrated in the genome of the host or may be expressed from a non-integrating element.

The term "increased expression", as used herein, means that the amount of a protein of interest expressed in a host organism having decreased Galectin-1 gene expression or decreased activity of the Galectin-1 gene product is increased compared to the amount of the same protein expressed in a host in which the Galectin-1 gene activity or the Galectin-1 gene product activity is not inhibited.

The term "protein of interest" or "POI", as used herein, relates to any protein that is expressed via recombinant expression.

The term "nucleic acid molecule" or "nucleic acid sequence", as used herein, relates to DNA (deoxyribonucleic acid) or RNA (ribonucleic acid) molecules. Said molecules may appear independent of their natural genetic context and/or background. The term "nucleic acid molecule/sequence" further refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms.

"At least one", as used herein, relates to one or more, in particular 1, 2, 3, 4, 5, 6, 7, 8, 9, 10 or more.

The term "sequence", as used herein, relates to the primary nucleotide sequence of nucleic acid molecules or the primary amino acid sequence of a protein.

The term "mammalian host cell", as used herein, relates to an organism that harbors the nucleic acid molecule or a vector containing the nucleic acid sequence encoding a protein of interest and having decreased expression of the Galectin-1 gene or decreased activity of the Galectin-1 gene product.

"Culturing", "cultivating" or "cultivation", as used herein, relates to the growth of cells in a specially prepared culture medium under supervised conditions. The term "conditions suitable for recombinant expression" relates to conditions that allow for production of the protein of interest in cells using methods known in the art, wherein the cells are cultivated under defined media and temperature. In this context, CO₂ conditions may be used which are known in the art or, optionally, the cell may be cultivated under CO₂-free conditions (e.g. MOPS buffer). The medium may be a nutrient, minimal, selective, differential, transport or enriched medium. Preferably, the medium is a nutrient medium. Growth and expression temperature of the mammalian host cell may range from 25 °C to 45 °C. Preferably, the growth and expression temperature range from 30 °C to 37 °C. The CO₂ culture and expression conditions may range from 2 % to 15 %. Preferably, the CO₂ culture and expression conditions range from 5 % to 10 %. Optionally, the CO₂ concentration can be dependent on the pH of the culture media, particularly when bioreactor cultivation is used. Conditions for such bioreactor cultivation are known in the art and comprise a pH ranging from 6.5 to 7.5.

The term "protein", as used herein, relates to one or more associated polypeptides, wherein the polypeptides consist of amino acids coupled by peptide (amide) bonds. The term polypeptide refers to a polymeric compound comprised of covalently linked amino acid residues. The amino acids are preferably the 20 naturally occurring amino acids glycine, alanine, valine, leucine, isoleucine, phenylalanine, cysteine, methionine, proline, serine, threonine, glutamine, asparagine, aspartic acid, glutamic acid, histidine, lysine, arginine, tyrosine and tryptophan.

The term "gene product", as used in the present invention, relates to a biochemical material, either RNA or protein, resulting from expression of a gene. Moreover, the proteins may form complexes with other proteins via covalent and non-covalent bonds.

The term "activity" or "protein activity" as interchangeably used herein relate to the capacity of a protein to catalytically react with substrates, to bind to other molecules, (e.g. DNA, RNA or other proteins) or to change its localization and in particular relates to its natural functionality. Different methods to measure each activity are known in the art.

The term "encoding a protein" means that the information of a gene sequence is converted into the corresponding peptide sequence information.

The term "inhibiting", as used herein, relates to a significant and detectable reduction of protein activity or gene expression activity caused by an effector molecule. Preferred inhibition activities resulting from protein activity or gene expression activity inhibition are more than 10%, 20%, 50%, 80% or 95%.

The terms "Galectin-1 gene" or "Lgals-1", as used interchangeably herein, relate to a nucleic acid sequence encoding a Galectin-1 protein. The nucleic acid sequence of the Galectin-1 gene from *Cricetulus griseus* is set forth in SEQ ID NO:1.

"RNA" or "ribonucleic acid" as interchangeably used herein relates to a chain of nucleotides wherein the nucleotides contain the sugar ribose and bases selected from the group of adenine (A), cytosine (C), guanine (G), or uracil (U). "DNA" or "deoxyribonucleic acid" as interchangeably used herein relates to a chain of nucleotides wherein the nucleotides contain the sugar 2'-deoxyribose and bases selected from adenine (A), guanine (G), cytosine (C) and thymine (T).

The term "RNAi" as used herein relates to RNA interference (RNAi) which also called post transcriptional gene silencing (PTGS) and is a biological process in which RNA molecules inhibit gene expression, typically by causing the destruction of specific mRNA molecules.

The term "siRNA" or "small interference RNA" as interchangeably used relates to a class of double-stranded RNA molecules, 19-25 base pairs in length. siRNA plays many roles, but its most notable is in the RNA interference (RNAi) pathway, where it interferes with the expression of specific genes with complementary nucleotide sequence. siRNA also acts in RNAi-related pathways, e.g., as an antiviral mechanism or in shaping the chromatin structure of a genome.

The term "shRNA" or "small hairpin RNA" relates to a sequence of RNA that makes a tight hairpin turn that can be used to silence target gene expression via RNA interference (RNAi). Expression of shRNA in cells is typically accomplished by delivery of plasmids or through viral or bacterial vectors. Preferably, the promoter of choice is a DNA-dependent RNA polymerase III promoter. shRNA is an advantageous mediator of RNAi in that it has a relatively low rate of degradation and turnover.

The term "genome", as used herein, relates to the entirety of an organism's hereditary information. It is encoded either in DNA or, for many types of viruses, in RNA. The genome includes both the genes and the non-coding sequences of the DNA/RNA.

The term "stably integrated into the genome" means that a nucleic acid sequence which was transfected into a mammalian host cell (and may comprise the sequence of an shRNA) is covalently linked on its ends with the DNA of the mammalian host cell allowing replication of this sequence during cell division.

"CHO cell" or "Chinese hamster ovarian cell" as used interchangeably relate to a cell line derived from the ovary of the Chinese hamster (*Cricetulus griseus*). CHO cells are epithelial cells which grow as an adherent monolayer or in suspension. They, characteristically, require the amino acid proline in their culture medium. Different subgroups of CHO cells are CHO DP-12 cells, CHO-K1 cells, CHO/dhfr- cells, CHO-S cells, CHO-GS cells CHO-K1 DUX B11 cells (Simonsen and Levinson (1983), PNAS, 80, 2495-2499), dp12.CHO cells (EP 307,247), CHO pro3⁻ cells and CHO-DG44 cells. In a preferred embodiment of the present invention the mammalian host cell is a CHO-DP12 cell containing the p6G4V11-N35E.choSD.10 vector (ATCC CRL-12445). In other preferred embodiments of the invention the CHO cell is CHO pro-, CHO S, CHO WTT (WT-1, 2, 3, 4 or 5), CHO pro-3, CHO pro-3 MtxRI, RII or RIII, CHO UA21, CHO DG21 or DG22, CHO UA41, CHO DG41, 42, 43, 44 or 45, CHO DR1000L-4N, CHO DG44 suspension, CHO GAT-, CHO SC1, CHO AA8, CHO K1, CHO K1SV, CHO UKB25 (d+/d-), CHO DUK-B11(d+/d-), CHO DUK22(d-/d-), CHO DUK51(d-/d-), CHO DXA11, DXB11, DXC11, DXE11, DXF11, DXG11, DXH11, DXI11 or DXJ11, CHO-T, CHO 3E7 or freestyle CHO-S.

"Antibody", also known as an immunoglobulin (Ig), as used herein relates to a large Y-shaped protein that is used by the immune system to identify and neutralize foreign objects such as bacteria and viruses. Antibodies are typically made of basic structural units - each with two large heavy chains and two small light chains. There are several different types of antibody heavy chains, and several different kinds of antibodies, which are grouped into different immunoglobulin isotypes based on which heavy chain they possess. Five different antibody isotypes are known in mammals, which perform different roles, and help direct the appropriate immune response for each different type of foreign object they encounter. In a preferred embodiment of the present invention, the recombinantly expressed protein is an IgG. Antibodies are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "fed-batch process", as used herein, relates to a process which is based on feeding of a growth limiting nutrient substrate to a culture. The fed-batch strategy may be used to reach a high cell density in the bioreactor, to induce production and/or to enhance the productivity. In a preferred embodiment the feed solution is highly concentrated to avoid dilution of the bioreactor. The controlled addition of the nutrient directly affects the growth rate of the culture and helps to avoid overflow metabolism (e.g. formation of lactic acid in cell cultures).

"Kit", as used herein, relates to a kit-of-parts wherein the separate components of the kit are physically separated as individual components.

The inventors of the present invention have unexpectedly found that inhibition of the expression of the Galectin-1 gene or of the activity of the Galectin-1 gene product in a mammalian host cell results in an increased expression of a recombinantly expressed protein of interest. In a preferred embodiment, the Galectin-1 gene is inhibited by shRNA. In a further preferred embodiment, the mammalian host cell is a CHO cell and is cultured by fed-batch process.

A first aspect of the invention relates to a method for the recombinant expression of a protein of interest in a mammalian host cell, wherein the method comprises culturing the mammalian host cell comprising a nucleic acid sequence encoding the protein of interest under conditions suitable for recombinant expression of the protein of interest, and inhibiting the expression of the Galectin-1 gene or the activity of the Galectin-1 gene product in the mammalian host cell.

In specific embodiments of the invention, the mammalian cell is a CHO cell (Chinese hamster ovarian), a CAP cell (human aminocyte), a PER.C6 cell (human retina), a NSO or Sp2/0 cell (mouse myeloma), an EB66 cell (duck), a BHK cell (hamster), a freestyle HEK 293-F, a HEK 293 6E or a HEK 293T cell (human embryonic kidney) or a CAP-T cell (human aminocyte). In other specific embodiments of the invention, the mammalian host cell is an epithelial cell. In more preferred embodiments, the mammalian host cell is a Chinese hamster ovarian (CHO) cell. CHO cells comprise cell lines that are originally isolated by Tjio and Puck {Tjio, H.J. et al. (1958), Journal of experimental medicine, 108, 259-271} or cell lines that were derived from these original cells and comprise point mutations, deletions of nucleic acid sequences or integration of additional nucleic acid sequences relative to the original CHO cell line. The group of CHO cells may include, but is not limited to CHO DP-12 (ATCC CRL-12445) cells, CHO-K1 (ATCC CCL-61) cells, CHO/dhfr- (ATCC CRL-9096) cells, CHO-S (Schifferli,K. et al. (1999), Focus, 21, pages 16-17), CHO-GS (Bebbington, C.R. et al. (1992), Biotechnology, 10, pages 169-175), CHO-K1 DUX B11 (Simonsen and Levinson (1983), PNAS, 80, 2495-2499), dp12.CHO (EP 307,247), CHO pro3⁻ and CHO-DG44 cells {Urlaub et al. (1983) Cell, 33, 405-412}. In a preferred embodiment, the mammalian host cell is a CHO DP-12 cell.

In further embodiments, the cells are cultured as an adherent cell monolayer or being suspended in the culture media. Preferably, the mammalian host cell may be cultured in suspension. Cultivation methods to grow the mammalian host cell to express a protein of interest are batch cultivation, perfusion or fed-batch cultivation. Said methods are known in the art. In a further specific embodiment, the mammalian host is cultured by fed-batch process. More preferably, the cultivation is a discontinuous fed-batch process.

In specific embodiments of the invention, the Galectin-1 gene or its gene product are inhibited by RNAi, anti-Galectin-1 antibodies, small molecule inhibitors or gene deletion, preferably gene deletion via zinc-finger nucleases (ZNF). Preferred inhibition activities of RNAi, anti-Galectin-1 antibodies or small molecule inhibitors are be less than 35 %, 30 %, 25 %, 20 %, 15 %, 10 % or 5 % of the activity measured in untreated cells. In other specific embodiments of the invention, the Galectin-1 gene product is inhibited by lactose or lactose derivatives. The preferred Galectin-1 activity after lactose or lactose derivative treatment may be less than 35 %, 30 %, 25 %, 20 %, 15 %, 10 % or 5 % of the activity measured in untreated cells. In order to measure the Galectin-1 activity β-galactoside binding assays or cell aggregation assays may be used. Such assays are known in the art {Iurisci, I. et al. (2009) Anticancer research, 26, 403-410}.

In further specific embodiments of the invention, the RNAi is shRNA, siRNA, miRNA (microRNA) or miRNA adapted shRNA. The preferred Galectin-1 expression remaining after treatment with RNAi may be less than 35 %, 30 %, 25 %, 20 %, 15 %, 10 % or 5 % of the Galectin-1 expression measured in control cells (cells that are transfected with a control RNA or a control vector). The inhibition of Galectin-1 gene expression can be measured by using qRT-PCR or micro-array analysis. Quantitative real-time PCR (qRT-PCR) allows both, detection and quantification, of nucleic acid molecules. It is known in the art that real-time PCR measurements can be combined with reverse transcription to quantify messenger RNA and non-coding RNA. Protocols for measurements via RT-PCR are described by VanGuilder et al. {VanGuilder, H.D. et al. (2008) Biotechniques, 44, 619-626}. Micro-array protocols to quantify mRNA are described by Suarez et al. {Suarez, E. (2009) Puerto Rico health sciences journal, 28, 89-104}.

In order to produce nucleic acid vectors containing nucleic acid sequences encoding shRNA or a protein of interest cloning techniques may be required. Several cloning techniques, including amplification of nucleic acids, their restriction by according enzymes, purification and ligation, and transformation techniques, are known in the art and described in more detail by Sambrook et al.{Sambrook, J. et al. (1989) Molecular Cloning: A Laboratory Manual, 2nd Ed., Cold Spring Harbor Laboratory Press, Cold Spring Harbor, NY}. The produced nucleic acid constructs are verified by sequencing. Sequencing of the nucleic acid constructs can be done by the chain termination method, Sanger sequencing or Maxam-Gilbert sequencing or any other technique known in the art. Alternatively, high-throughput sequencing, like pyrosequencing, SOLiD sequencing or DNA nanoball sequencing, is used to determine the sequence of the nucleic acid molecules {Alphey, L. (1997) DNA Sequencing: From Experimental Methods to Bioinformatics, 1st Ed., Bios Scientific Pub Ltd., Oxford, UK}.

Vectors encoding shRNA or a protein of interest or siRNA molecules may be transfected by different methods, such as electroporation, calcium-phosphate transfection or by the assistance of cationic lipids. Alternatively, vectors encoding shRNA or a protein of interest may be inserted into the mammalian host cell by viral transduction. Protocols to insert vectors or siRNA into a target cell are known in the art.

In specific embodiments of the invention, vectors encoding shRNA are integrated in the genome of the mammalian host cell. Integrative vectors and protocols of nucleic acid integration in target genomes are known in the art {Gad, S.C. (2007) Handbook of pharmaceutical biotechnology, John Wiley & Sons, New Jersey, USA}. Nucleic acid integration can be detected via southern-blotting.

Increased expression of a protein of interest may be measured by different methods, such as enzyme-linked immuno sorbent assay (ELISA), spectrometric assays, enzyme activity-based assays, protein-arrays or HLPC. In preferred embodiments, the expression of a protein of interest in a mammalian host comprising the inhibition of the expression of the Galectin-1 gene or of the activity of the Galectin-1 gene product is increased more than 20 %, 40 %, 60 %, 80 % or 100 % compared to the expression of the protein of interest in the same mammalian host cell that is transfected with a control RNA or a control vector.

In specific embodiments, the recombinantly expressed protein of interest can be purified by methods known in the art. These methods include, but are not limited to chromatography or ultracentrifugation.

In another aspect, the invention relates to a use of an shRNA or an siRNA directed against the Galectin-1 gene for increasing the expression of a protein of interest in a mammalian host cell. The above described embodiments relating to the method for recombinant expression of a protein of interest may also relate to the use of an shRNA or an siRNA directed against the Galectin-1 gene for increasing the expression of a protein of interest.

In a further aspect, the present invention relates to a kit comprising a CHO cell and an shRNA or an siRNA, wherein the shRNA or the siRNA inhibit the expression of the Galectin-1. Embodiments related to the method for recombinant expression of a protein of interest may also relate to the kit of the invention.

The present invention is further illustrated by the following examples. However, it should be understood, that the invention is not limited to the exemplified embodiments.

### EXAMPLES

### Example 1: Construction of stable pLVX-shRNA1 CHO DP-12 cell pools

In order to analyze recombinant protein expression in Chinese Hamster ovary cells (CHO) CHO DP-12 cells were chosen as a model system. The CHO DP-12 cell pool was generated by transfection of CHO cells with the p6G4V11-N35E.choSD.10 vector and subsequent selection on methotrexate (MTX). This expression vector encodes the sequence of the heavy and light chain of the monoclonal murine 6G4.2.5 antibody (US patent No. 6,133,426 and EP patent No. 1415998) and allows recombinant expression of the murine antibody in the transfected cells.

For knockdown experiments the candidate genes Set, Bad and Lgals-1 (Galectin-1) were chosen. siRNA sequences for Set, Bad and Lgals-1 were calculated by either the siRNA Selection Program {Yuan et al. (2004), Nucleic acids research 32, 130-134} or the Ambion siRNA Finder. In order to identify the correct Set nucleic acid sequence that may be used as a template sequence to determine the siRNA of Set, the RNA of CHO DP-12 cells was purified and the Set gene was sequenced. The murine Bad nucleic acid sequence has been used as the template sequence to calculate the Bad siRNA sequence for knockdown in CHO DP-12 cells. The Lgals-1 nucleic acid sequence of CHO cells has been disclosed by Becker et al. {Becker, J. et al. (2011), Journal of Biotechnology, 156, 227-235}. This sequence was used to generate the corresponding siRNA. The shRNA and siRNA sequences used for Set (SEQ ID Nos. 7 and 8), Bad (SEQ ID Nos. 11 and 12) and Lgals-1 (SEQ ID Nos. 3 and 4) knockdown experiments are shown in the sequence listing.

In order to generate CHO cells exhibiting persistent knockdowns of Set, Bad and Lgals-1, the above described shRNA sequences were cloned into the multiple cloning site of the pLVX-shRNA1 transfervector (Takara Bio Europe/Clontech, France) via EcoRI/BamHI digestion and subsequent ligation.

The above plasmids were used to generate corresponding lentiviruses for transduction of CHO DP-12 cells. The lentiviruses that carry the different transfervectors were generated in HEK293FT cells by using the Lenti-X™ Lentiviral Expression System (Takara Bio Europe/Clontech, France) in accordance with the protocol of the manufacturer. After 72 h the virus containing supernatant was centrifuged for 10 min at 200 g and 4 °C. Afterwards, the supernatant of the centrifugation step was purified by passage through a 0.45 µm filter (Sartorius AG, Göttingen). Before adding the virus containing supernatant to the CHO DP-12 cells, the cells were treated with 4 µg/ml polybrene (hexadimethrinbromide, Sigma-Aldrich, USA). The transduction was carried out at 33 °C for 5 h. After a two-times wash with PBS and 48 h after starting the transduction process cells were selected at 37 °C by using 5 µg/ml puromycin. The selection step was completed after 12-14 days.

### Example 2: Persistent knockdown of Set, Bad and Lgals-1 in CHO DP-12 cells

The CHO DP-12 cells containing the integrated nucleic acid sequences of the control vector or Set, Bad and Lgals-1 shRNA sequences were cultured in TC42 (TeutoCell AG, Bielefeld, Germany) media supplemented with 5 mM glutamine, 200 nM MTX and 100 ng/l IGF. For optimal growth the cells were kept on a shaker using 185 rpm under conditions of 5 % CO₂ and 80 % humidity at 37 °C. For fed-batch experiments the starting volume of 20 ml TC42 medium including 5 mM glutamine was inoculated with 5·10⁵ cells/ml. The feed started on day 2 and was increased until day 7. Since day 7 the feed was given in constant amounts (see below Table). As feed TCx2D (TeutoCell AG, Bielefeld, Germany) was used which was supplemented with 20 g/l glucose and 5.5 g/l glutamine. Samples were taken at different timepoints for Cedex measurements and for product analytics.

**Table 1**

| | | | | | | | |
|---|---|---|---|---|---|---|---|
| Cultivation time [d] | 1 | 2 | 3 | 4 | 5 | 6 | 7 |
| Feed amount [ml/20 ml] | 0 | 0.4 | 0.8 | 1.2 | 1.6 | 2 | 2.4 |

To measure the knockdown of Set, Bad and Lgals-1 genes in CHO DP-12 cells quantitative real-time PCR (qRT-PCR) analysis was used. In a first step, the RNA of the CHO cells was purified either by using the NucleoSpin^{®} RNA II kit (Macherey-Nagel, Düren, Germany) according to the manufacturer's protocol or by following the one-step purification protocol of Chomczynski and Sacchi {Chomczynski, P. et al. (1987) Analytical Biochemistry, 162, 156-159) using TRIzol^{®} (Life Technologies GmbH, Darmstadt, Germany). The purified RNA was used as a template to synthesize complementary DNA (cDNA). cDNA has been generated utilizing the RevertAid™ First Strand cDNA Synthesis Kit (Fermentas, St. Leon-Rot, Germany) according to the manufacturer's protocol. RT-PCR primer pairs to amplify Set, Bad and Lgals-1 genes are set forth in the sequence listing (SEQ ID Nos. 5, 6, 9, 10, 13 and 14).

For qRT-PCR analysis the LightCycler^{®} 480 (Roche Diagnostics, Mannheim, Germany) was used in combination with the Platinum^{®} SYBR^{®} Green qPCR SuperMix-UDG Kit (Life Technologies GmbH, Darmstadt, Germany). The manufacturer protocols were essentially followed with the exception that the reaction volume was reduced from 50 µl to 30 µl. All reactions have been measured in triplicates. Normalization of gene expression was based on up to three housekeeping genes. The housekeeping genes providing the standard for normalizazion were β-actin (*Aktb*), Vezatin (*Vezt*) and glyceraldehyde 3-phosphat dehydrogenase (*Gapdh*)*.* The according qRT-PCR primers (SEQ ID Nos. 15-20) are shown in the sequence listing.

Set knockdown (Set-kd) CHO DP-12 cells were tested for their expression of the Set gene under fed-batch culture conditions. The levels of Set mRNA have been determined in CHO DP-12 cells, CHO DP-12 cells containing the control vector (pLVX cells) and Set-kd cells. The primer pair for Set quantification is shown in the sequence listing (SEQ ID Nos. 9 and 10). Quantitative RT-PCR analysis revealed that the expression of Set mRNA in Set-kd CHO DP-12 cells has been reduced to about 8 % of the expression observed in non-transduced cells and to about 10 % compared to the vector control cell pool (Figure 1).

Quantitative RT-PCR analysis of Bad expression under fed-batch conditions has been measured by using the RT-PCR detection primer SEQ ID Nos. 13 and 14. CHO DP-12 cells, pLVX cells and Bad-kd cells have been analyzed. A reduction of about 14 % of expression compared to CHO DP-12 cells was observed for Bad-kd CHO DP-12 cells. Comparison between Bad-kd cells and pLVX cells revealed a Bad knockdown of about 10 % (Figure 2).

Levels of Lgals-1 mRNA expression were investigated under fed-batch conditions in CHO DP-12 cells, CHO DP-12 cells containing the vector control and Lgals-1-kd cells using the detection primer pair of SEQ ID Nos. 5 and 6. This analysis demonstrated that the expression of Lgals-1 mRNA in Lgals-1-kd CHO DP-12 cells has been reduced to about 7 % of the expression observed in CHO DP-12 cells. Comparison between Lgals-1-kd cells and pLVX cells indicates that the expression of Lgals-1 mRNA was reduced to about 10 % (Figure 3).

### Example 3: Determination of the number and density of viable CHO DP-12 cells, pLVX cells, Set-kd cells, Bad-kd cells and Lgals-1-kd cells

Set-kd, Bad-kd and Lgals-1-kd CHO DP-12 cells were cultured under fed-batch conditions as described in Example 2. To determine the number and density of viable cells samples of each cell pool were analyzed with the cell density examination system Cedex (Roche Innovatis, Mannheim, Germany). Samples of the cells were mixed with trypan blue and measured by Cedex. Trypan blue can enter dead cell which have damaged cell membranes to stain these cells dark blue {Tennant, J.R. (1964) Transplantation, 2, 685-694}. The Cedex software can recognize the stained cells on pictures taken with a CCD camera.

Until day 0.7 all cells, namely the CHO DP-12 cells, the pLVX cell pool, the Set-kd cell pool, the Bad-kd cell pool and the Lgals-1-kd cell pool showed identical growth (Figure 4). At this time point the feed started. After one more day (day 1.7) the densities of viable cells began to change among the different cell pools. Until the start of their death phase (day 8.8) the Lgals-1-kd cells continuously had the highest viable cell density. This cell pool reached its maximum viable cell density on day 7.7 with 203·10⁵ cells/ml. After day 8.8 the density of viable Lgals-1-kd cells dropped faster compared to the other cell pools. Therefore, the requirement to stop the fed-batch cultivation (a viable cell density of 40 % or less) was reached one day earlier than in the other cell pools.

The Bad-kd cells have a similar density curve as the Lgals-1-kd cells. However, their density of viable cells was always less than the one of Lgals-1-kd cells. For both cell pools the growth rate started to slow down on day 5.7. Bad-kd cells reached their highest density on day 7.7 with a maximum of 166·10⁵ cells/ml. The Set-kd and pLVX cells showed an almost similar cell density over 14 days. These two cell pools revealed increased growth compared to the non-transduced CHO DP-12 cell pool.

Figure 5 shows the corresponding cell numbers of the different cells after normalization to the volume of the fed-batch culture.

### Example 4: Calculation of the growth rate and cell-time integral for CHO DP-12 cells, pLVX cells, Set-kd cells, Bad-kd cells and Lgals-1-kd cells

The calculations for the maximum and average growth rate and the cell-time integral of the different CHO cell lines were based on the data obtained in the above described fed-batch experiment (Example 2 and Example 3). In order to calculate the growth rate parameters, formulas as described by Pirt {Pirt, S.J. (1985) Principles of microbe and cell cultivation, Blackwell Science Ltd.} were used. The calculated maximum growth rates of the Set-kd cells, Bad-kd cells and Lgals-1-kd cells are almost similar (between 0.83 and 0.85) and higher than the maximum growth rate of the CHO DP-12 cells and the pVLX CHO cells (Figure 6). Moreover, the average growth rate of Lgals-1-kd cells (0.70) was only slightly higher than the one of Bad-kd cells (0.69) (however, the maximum cell density of Lgals-1-kd cells was significantly higher than the cell densities measured in the other cell pools).

The cell-time integral describes the area under the cell density curve of each cell type. Due to the fact that changes in culture volume are normalized in the calculation, the dimension of the cell-time integrals is indicated in cells multiplied by time (days). Comparison of the cell-time integral of the pLVX cells and the Set-kd cell pool, namely 226·10⁷ cells·d and 220·10⁷ cells.d, revealed almost similar results. Thus, the cell-time integral of these cells were 48.5 % and 44.4% higher compared to CHO DP-12 cells. The cell-time integrals of the Bad-kd and Lgals-1-kd cell pools showed higher values than the other investigated cells. With reference to the non-transduced CHO DP-12 cells, the Bad-kd and Lgals-1-kd cell pools had 84.2 % and 92.2 % higher integrals. Therefore, the yield of viable cells for these two cell pools during fed-batch culture experiments was almost twice as high as the yield of CHO DP-12 cells.

### Example 5: Determination of the product titer of recombinantly expressed antibodies

The CHO DP-12 cells, the pVLX cell pool, the Set-kd cell pool, the Bad-kd cell pool and the Lgals-1-kd cell pool were fed-batch cultured as described in Example 2. Cell-free samples from culture media were taken at different time points. These samples contained the recombinantly expressed 6G4.2.5 antibody. To determine the product titer of the antibodies expressed in the different cells a protein A column (POROS A, Life Technologies GmbH, Darmstadt, Germany) and a HPLC system were used. Before this analysis was carried out, 120 µl of the samples were centrifuged for 1 min at 4 °C (Hereaus fresco, Thermo Fisher Scientific, Schwerte, Germany) through a 0.2 µm centrifugal filter. 100 µl of the filtered sample were transferred into a fresh tube. The HLPC analysis provided peak areas that were proportional to the antibody concentration. The antibody concentration was then calculated with support of internal standards.

Figure 8 shows the product concentration of antibodies that were recombinantly expressed in the CHO DP-12 cells, the pVLX cell pool, the Set-kd cell pool, the Bad-kd cell pool and the Lgals-1-kd cell pool. The lowest product titer was observed in the CHO DP-12 reference culture (176 mg/l). This value was measured on day 7.7, thus indicating a corresponding relationship between cell growth and antibody synthesis. At the end of fed-batch cultivation the product titers of all cell lines and cell pools were decreasing. The reason for this product concentration decrease may be related to degradation (e.g. hydrolysis) and/or digestion by proteases originating from damaged cells. The measurement of the antibody concentration produced by the pVLX cell pool, the Set-kd cell pool and the Bad-kd cell pool demonstrated a similar antibody synthesis for these cell pools. The maximum product titers of these cells were observed on day 8.8 or 9.8 with values that were ranging from 238 mg/l to 253 mg/l. In contrast, the maximum product titer of the Lgals-1-kd cell pool was significantly increased over the results obtained for CHO DP-12 cells and the pLVX cell pool. The maximum titer was reached on day 8.8 showing 456 mg/l. This antibody concentration relates to a 159.1 % increase over the reference culture CHO DP-12 and to a 91.6 % increase over the pLVX cell pool.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. Other embodiments are within the following claims. In addition, where features or aspects of the invention are described in terms of Markush groups, those skilled in the art will recognize that the invention is also thereby described in terms of any individual member or subgroup of members of the Markush group.

One skilled in the art would readily appreciate that the present invention is well adapted to carry out the objects and obtain the ends and advantages mentioned, as well as those inherent therein. Further, it will be readily apparent to one skilled in the art that varying substitutions and modifications may be made to the invention disclosed herein without departing from the scope and spirit of the invention. The compositions, methods, procedures, treatments, molecules and specific compounds described herein are presently representative of preferred embodiments are exemplary and are not intended as limitations on the scope of the invention. Changes therein and other uses will occur to those skilled in the art which are encompassed within the spirit of the invention are defined by the scope of the claims. The listing or discussion of a previously published document in this specification should not necessarily be taken as an acknowledgement that the document is part of the state of the art or is common general knowledge.

The invention illustratively described herein may suitably be practiced in the absence of any element or elements, limitation or limitations, not specifically disclosed herein. Thus, for example, the terms "comprising", "including," containing", etc. shall be read expansively and without limitation. The word "comprise" or variations such as "comprises" or "comprising" will accordingly be understood to imply the inclusion of a stated integer or groups of integers but not the exclusion of any other integer or group of integers. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by exemplary embodiments and optional features, modification and variation of the inventions embodied therein herein disclosed may be resorted to by those skilled in the art, and that such modifications and variations are considered to be within the scope of this invention.

The content of all documents and patent documents cited herein is incorporated by reference in their entirety.

## Claims

1. Method for the recombinant expression of a protein of interest in a mammalian host cell, comprising:
culturing the mammalian host cell comprising a nucleic acid sequence encoding the protein of interest under conditions suitable for recombinant expression of the protein of interest, and
inhibiting the expression of the Galectin-1 gene or the activity of the Galectin-1 gene product in the mammalian host cell.

2. The method of claim 1, wherein the expression of the Galectin-1 gene is inhibited by RNAi.

3. The method of claim 2, wherein the RNAi is shRNA or siRNA.

4. The method of claim 3, wherein the shRNA comprises the nucleic acid sequence set forth in SEQ ID NO:3.

5. The method of claim 3, wherein the siRNA comprises the nucleic acid sequence set forth in SEQ ID NO:4.

6. The method of any one of claims 1 to 5, wherein the mammalian host cell is a Chinese hamster ovarian (CHO) cell.

7. The method of any one of claims 1 to 6, wherein the mammalian host cell is a Chinese hamster ovarian (CHO) DP-12 cell (ATCC CRL-12445).

8. The method of any one of claims 1 to 7, wherein the protein of interest is an antibody.

9. The method of claim 8, wherein the antibody is the monoclonal murine 6G4.2.5 antibody (ATCC-HB-11722).

10. The method of any one of claims 1 to 9, wherein the mammalian host cell is cultured by fed-batch process.

11. The method of any one of claims 2 to 10, wherein a nucleic acid sequence encoding the RNAi is stably integrated into the genome of the mammalian host cell.

12. A kit comprising a CHO cell and an shRNA or an siRNA, wherein the shRNA or the siRNA inhibit the expression of the Galectin-1.

13. The kit of claim 12, wherein the shRNA comprises the nucleic acid sequence set forth in SEQ ID NO:3 or the siRNA comprises the nucleic acid sequence set forth in SEQ ID NO:4.

14. Use of an shRNA or an siRNA directed against the Galectin-1 gene for increasing the expression of a protein of interest in a mammalian host cell.

15. The use of an shRNA according to claim 13, wherein the shRNA comprises the nucleic acid sequence set forth in SEQ ID NO:3 or the siRNA comprises the nucleic acid sequence set forth in SEQ ID NO:4.
